# EUROPEAN PATENT APPLICATION

(11) **EP 2 072 616 A1**
(43) Date of publication of application: **24.06.2009**
(21) Application number: 07123399.3
(22) Date of filing: 17.12.2007
(51) Int. Cl.: C12N 5/06, C12N 5/10

(54) **Method for reprogramming in vitro stem cells and somatic cells into germinal cells**

(71) Applicant: Institut National De La Recherche Agronomique, 75007 Paris (FR)
(72) Inventor: Pain, Bertrand, 63100, CLERMONT FERRAND (FR); Samurat, Jacques, 69003, LYON (FR); Lavial, Fabrice, 42100, SAINT ETIENNE (FR); Rouault, Jean-Pierre, 69400, VILLEFRANCHE S/SAONE (FR); Bouhaillier, Frantz, 69003 Lyon (FR)
(74) Representative: Tetaz, Franck Claude Edouard

(57) **Abstract**

The present invention is related to a method for inducing *in vitro* the differentiation of stem cells or somatic cells towards germinal cells, comprising the following steps :
• cultivating said stem cells or somatic cells in a medium allowing their differentiation, and
• collecting the germinal cells from the culture medium,

wherein said cells are cells transformed with an exogenous genetic construction comprising at least a Vasa gene.
Germinal cells such as obtained and chimeric animals are also an object of this invention.

## Description

### INTRODUCTION

The present invention relates to a method for producing *in vitro* germinal cells from stem cells or somatic cells. The invention is also related to germinal cells such as obtained. Preferentially these cells express a transgene of interest. Injection of these germinal cells into a receptor embryo leads to the colonization of the gonads of said embryo, and therefore allows the generation of chimeric animals expressing a transgene of interest into their germinal cells. Therefore, the chimeric animals are able to transmit the transgene to their offspring.

### BACKGROUND ART

*Vasa* gene expression was determined as being one of the most specific markers for germ cells (Tanaka et al., 2000; Extavour and Akam, 2003). Initially identified in Drosophila, this gene encodes a DEAD box RNA binding protein with an ATP-dependent RNA helicase activity, homologue to the transcription factor *eIF4* (Lasko et al., 1988). It is sometimes called DDX4.

This gene and its specificity are highly conserved all along the metazoans (Mochizuki et al., 2001) and found in Human (Castrillon et al., 2000), mouse (Fujiwara et al., 1994), Xenopus (Komiya et al., 1994), zebrafish (Yoon et al., 1997; Brat et al;, 2000), but also in brachiopod (Sagawa et al;, 2005), oyster (Fabioux et al., 2004), planaria (Shibata et al., 1999), hydra (Mochizuki et al., 2001), polyaechetes Platynereis and in C. elegans (Shisa et al., 2001).

A Chicken Vasa homologue (*Cvh*) was identified and cloned in year 2000 (Tsunekawa et al., 2000).

Germ cells in chicken are predetermined very early during development and marked by the specific marker Vasa (Tsukenawa et al., 2000; Extavour and Akam, 2002). These cells are called Primordial Germ Cells and can be isolated and cultured *in vitro* (van de Lavoir, Nature, 2006). These cells are also able to differentiate into somatic cells.

In chicken, *Cvh* positive cells were detected as early as the first cleavages and around 30 cells are present in stage X (EG & K) embryos, usually observed in the freshly laid egg. About 45 to 60 positive cells were identified at later stage 2-3 (H&H) on the hypoblast layer and 200 to 250 positive cells at stage 4 (H&H) located anteriorly in the germinal crescent as a consequence of morphogenetic movements (Tsunekawa et al., 2000).

Primordial Germ Cells can be derived from germinal crests, collected in embryos in formation. These cells can be maintained *in vitro*; however they have a different epigenic situation of embryonic stem cells (Shiota et al., 2001; Reik et al., 2001)

More information on the pattern of markers specific expression during embryogenesis in chicken is disclosed in patent application PCT/EP2007/052574, related to a combination of molecular markers useful to identify the nature of chicken embryonic cells.

In mammals, similar mechanisms are observed. Germinal cells of the mouse are derived from a founder population of primordial germ cells that are set aside early in embryogenesis. Primordial germ cells arise from the proximal epiblast, a region of the early mouse embryo that also contributes to the first blood lineages of the embryonic yolk sac.

Chicken Embryonic Stem Cells (cESC) arose from the *in vitro* culture of pre-primitive streak (stage IX-XII) blastodermal cells. They were characterised by the presence of typical ESC markers such as alkaline phosphatase (AP), telomerase activity and reactivity toward different specific antibodies including ECMA-7, SSEA-1 and SSEA-3 (Pain et al., 1996; patent application FR 94/12598; Pettite et al., 2004). As for their mammalian counterpart, these cells are pluripotent and able to give rise to differentiated derivatives from the three germ layers both *in vitro* and *in vivo* (Pain et al., 1996; Van de Lavoir et al., 2005).

However, the germinal behaviour gets lost quickly after maintenance of cells *in vitro. In vivo,* upon injection in recipient embryos, ES cells can form somatic chimera but are not able to colonize efficiently the gonads of host embryos.

In contrast, non cultured blastodermal cells and long term cultured chicken Primordial Germ Cells maintain their germ line competency. Isolation of PGC *in vitro,* based on the cellular screening of cells expressing the Vasa marker, has been performed with success.

To produce chimeric and transgenic animals, it is primordial to obtain cells that can be manipulated *in vitro.* However, Primordial Germ cells can not be transformed with exogenous DNA as easily as ES cells. In particular, the efficiency of transfection using lipofection derived agents (such as Lipofectamin, lipofectamin 2000, (Invitrogen R), Fugene (Roche), PEI...) is rather low comparatively to the efficiency obtained in transfection of ES cells. Moreover the use of selection drugs such as Neomycine (G418), Hygroycine, puromycine, is also more efficient on ES cells comparatively to PGCs, since said PGCs proliferate slower than ES cells. I*n vitro,* stable clones were easier to obtain from ES cells rather than from PGCs.

Therefore, culture and isolation of ES cells that can be transformed and that can differentiate into germinal cells *in vitro* is primordial for creating transgenic germinal cells. These transgenic germinal cells would be used for colonizing gonads in a recipient embryo that would evolve in a chimeric animal, carrying a genetic modification that could be transmitted to the offspring.

In mouse and other mammals, the problem is not exactly the same.

Murine embryonic stem cells were first isolated in 1981, from the inner cell mass of blastocyst-stage embryos. They can be maintained in culture *in vitro* for many passages.

When injected into embryos, ESCs can contribute to tissues derived from all three germ layers and to the germline. Studies have successfully shown that ESCs can recapitulate features of embryonic development by spontaneously forming somatic lineages in culture.

Development of mouse primordial germ cells from mESC was observed after a 7 days culture in a non-supplemented medium (Hübner et al., Science, 2003; Geijsen, Nature, 2004). This spontaneous differentiation was also observed with human ESC.

It has also been shown that mouse ESCs can give rise to primordial germ cells in culture that are capable of undergoing meiosis and forming both male and female gametes.

However, the full potential of these ES-derived germ cells and gametes remains to be demonstrated.

Moreover, the proportion of such *in vitro*-obtained germinal cells is low, and cells have to be sorted and then concentrated to be amplified *in vitro* in a proliferative medium, before being submitted to the differentiation protocol in a specific differentiation medium with reduced concentration of serum and absence of cytokines and growth factors.

The use of successive steps of proliferation and differentiation of the transfected and sorted ES cells, to induce the final germ cell phenotype, is of primary importance.

Therefore, it appears that it does not exist in the prior art a simple and efficient way to obtain *in vitro* germinal cells from non-germinal cells.

### GENERAL DESCRIPTION OF THE INVENTION

The invention concerns a method for inducing *in vitro* the differentiation of stem cells or somatic cells, towards germinal cells, by genetically modifying these cells, and cultivating the transformed cells in an appropriate medium. Stem cells can be either embryonic or non-embryonic, including adult stem cells.

Inventors have discovered that chicken ES cells cultured *in vitro* can be induced into differentiation towards germinal cells, with a single genetic modification, consisting in the introduction into these cells of an exogenous gene *"Vasa".*

Mammalian embryonic stem cells can also be induced into differentiation towards germinal cells, with a genetic modification consisting in the introduction into these cells of an exogenous gene *"Vasa"* and a specific microRNA.

Moreover, the present invention offers a way to induce somatic cells from chicken or mammal into differentiation towards germinal cells, by transforming said cells with a genetic construction comprising both the gene *"Vasa"* and the specific microRNA.

The germinal cells obtained with the method of the invention show a characteristic expression pattern of markers specific of germinal cells; in particular chicken cells are characterized with the expression of specific markers such as described in the patent application PCT/ EP2007/052574.

Germinal cells obtained with the method of the invention are also characterized by the fact that they are able to colonize the gonads when they are injected into an embryo.

This method is in particular useful to produce transgenic germinal cells, and chimeric animals that can transmit the genetic modification to their offspring.

### DETAILLED DESCRIPTION

The present invention is related to a method for inducing *in vitro* the differentiation of stem cells or somatic cells towards germinal cells, comprising the following steps:
- cultivating said stem cells or somatic cells in a medium allowing their differentiation, and
- collecting the germinal cells from the culture medium,
wherein said stem cells or somatic cells are cells transformed with an exogenous genetic construction comprising at least a Vasa gene.

Cultivating cells in a medium allowing cell differentiation is known in the art. The person skilled in the art may choose an appropriate medium and/or appropriate growth conditions.

An appropriate medium is in particular a growth medium having a low percentage of serum, in particular less than 10% of serum. The man skilled in the art knows how to favorize the differentiation of the cells, by adding or deleting specific cytokines, growth factors and the like.

Particular media adapted for promoting the differentiation of chicken embryonic cells are presented in the examples below.

The step of collecting the differentiated germinal cells is well known by the man skilled in the art, and can be realized by several methods such as FACS cell sorting.

The terms "transformed" or "transformation" refer to the incorporation of exogenous nucleic acid into a cell, this acquisition of new genes being transitory (if the vector carrying genes is cured) or permanent (in the case the exogenous DNA is integrated chromosomally). These terms are used indistinctly with the terms "transfected" and "transfection". Cells having been transformed are also called "host cells".

Transformation of the cells can be realized by different techniques. The man skilled in the art knows how to choose the more appropriate technique for the cells that have to be transformed.

In particular, chicken ES cells can be transfected according to the method described in (Pain et al., 1996) and (Pain et al., 1999).

Murine ES cells can be transfected according to (Robertson et al., 1987) and as described in several independent protocols using either electroporation or lipofection.

Somatic Human Hela Cells (ATCC CCL 2), the well known somatic human cell line, can also be transformed according to the techniques well known by the man skilled in the art, such as phosphate calcium precipitates or lipofection with various lipofection agents provided by various companies (Roche, Invitrogen, BioRad, etc....).

The exogenous genetic construction according to the invention designates a cassette comprising, in the way 5' to 3', at least:
- an expression promoter functional in the transformed cells,
- a Vasa gene, including all or part of the coding sequence, but in all cases functional enough to obtain the differentiation of cells into germinal cells,
- a termination sequence functional in transformed cells.

All molecular biology techniques used for realizing the genetic construction are fully described in « Molecular cloning: a laboratory manual », 2nd edition, Cold Spring Harbor Laboratory Press, 1989, by Sambrook, Fritsch and Maniatis.

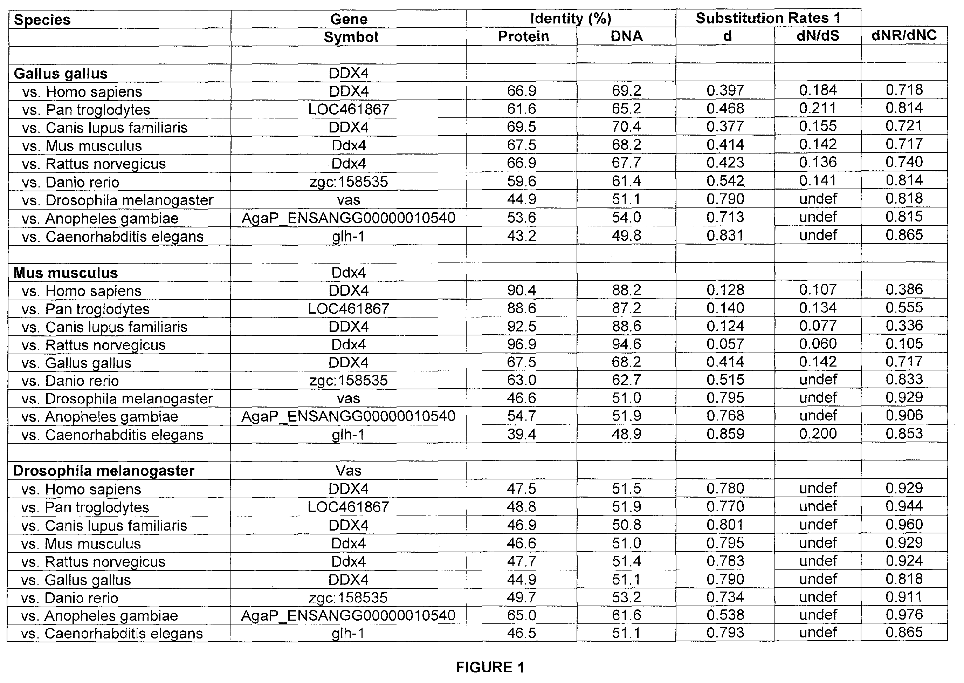

Used in the context of the present invention, the term "Vasa gene" means any gene or coding sequence from any species, encoding for a gene of the Vasa family. The phylogenetic tree of the Vasa family is presented in figure 1. More particularly it means any of the following:
- the Vasa gene 'DDX4' from the mouse (Fujiwara et al., 1994) with GenBank access N° D14859;
- the gene 'Cvh' from the chicken (Tsunekawa et al., 2000) with GenBank access N°AB004836;
- the Vasa gene of the rat (Komoya and Tanigawa, 1995) with GenBank access N°S75275;
- the Vasa gene of the Xenopus (Komiya et al., 1994) with GenBank access N° S69534;
- the Vasa gene from the zebrafish (Yoon et al., 1997) with GenBank access N° AB005147.

According to the present invention, the Vasa gene comprised in the cassette may be from the same species than the host cells, or from a different species than the host cells.

In particular stem cells or somatic cells can be transformed with a construction comprising:
- any Vasa gene from chicken, mouse, human, or any species, in particular a gene shown in the phylogenetic tree of figure 1, but also any gene belonging to the Vasa family and not shown here, or
- any variant having at least **50**% of homology with the DNA sequence of a Vasa gene, or a variant encoding for a protein having at least 30% of identity with the Amino Acid sequence of a Vasa-encoded protein.
   The invention is described in the Examples with respect to chicken Vasa gene. However, this designation has a more general meaning according to the invention, and covers the corresponding genes in other species. Using the GenBank references of the Vasa gene, those skilled in the art can determine equivalent genes in other species.
   Means of identification of the homologous sequences and their percentage homologies are well-known to those skilled in the art, and include in particular the BLAST programmes that can be used on the website http://www.ncbi.nlm.nih.gov/BLAST/ with the default parameters indicated on that website. The sequences obtained can be exploited (aligned) using for example the programmes CLUSTALW (http://www.ebi.ac.uk/clustalw/), with the default parameters indicated on these websites.
   The PFAM database (protein families database of alignments and hidden Markov models http://www.sanger.ac.uk/Software/Pfam/) is a large collection of alignments of protein sequences. Each PFAM makes it possible to visualise multiple alignments, view protein domains, evaluate distributions among organisms, gain access to other databases and visualise known protein structures.
   This method for obtaining differentiated germinal cells can be applied to different cellular types cultivated *in vitro.*
   In particular this method may be applied to chicken Embryonic Stem Cells (cESC), preferentially to cells that arose from the *in vitro* culture of pre-primitive streak (stage IX-XII) blastodermal cells. These cells may be obtained according to the technique described in (Pain et al., 1996) and (Pain et al., 1999).

Mammalian embryonic or adult stem cells can also be induced into differentiation towards germinal cells by using the method according to the invention. These cells are preferentially non-human. These cells are preferentially embryonic stem cells, and more preferentially murine embryonic stem cells. Murine ES cells have been extensively described in the scientific literature.

Surprisingly, the method according to the invention may also be applied to non-embryonic, somatic cells. Somatic cells include any cell forming a body, except germline cells. The term "somatic cells" include differentiated non-sex cells taken from a body for culture *in vitro,* and also immortalized non-sex cell lines maintained in culture. Somatic cells can be cells from any species and in particular human cells, murine cells, or chicken cells. An example of immortalized human cells is the cell line called "HeLa" (ATCC CCL-2).

In a preferred embodiment of the invention, cells and particularly somatic cells and mammalian stem cells, are transformed with a genetic construction comprising the human microRNA-34c, or a homolog sequence of such microRNA, presenting at least 90% of homology with the mature micro-RNA-34c, or a DNA sequence encoding for such miRNA or homolog.

MicroRNAs (miRNA) are single-stranded RNA molecules of about 21-23 nucleotides, which regulate gene expression. miRNAs are encoded by genes that are transcribed from DNA but not translated into protein, i.e. non-coding RNA; instead they are processed from primary transcripts known as *pre-miRNA* to short stem-loop structures called *pre-miRNA* and finally to functional miRNA. Mature miRNA molecules are partially complementary to one or more messenger RNA (mRNA) molecules, and their main function is to down regulate gene expression.

MicroRNAs have been initially identified in *Caenorhabditis elegans* as regulators of developmental timing (Lee et al. 1993; Bartel 2004), yet the term *microRNA* was only introduced in 2001 in a set of three articles in Science (26 October 2001). These RNAs have been identified in a broad range of animals, and some are conserved from *C. elegans* through *Drosophila melanogaster* to *Homo sapiens* (Pasquinelli et al. 2000; Lagos-Quintana et al. 2001; Lau et al. 2001; Lee and Ambros 2001).

The human microRNA-34c has been isolated from a regular screen using a panel of 200 miRNA (mirVana miRNA probe set, from Ambion) in order to identify microRNA that are expressed in a tissue-specific manner (Baskerville & Bartel, 2005). Different human tissues, at different stages of differentiation, were tested. Total low molecular weight RNA were obtained using protocols such as the FlashPage kit provided by Ambion (ref 131000) and the Rneasy Mini kit from Qiagen (ref 74104). The low molecular weight RNA, once isolated, were labelled and used for hybridization of the slides, according to the manufacturer.

Among the miRNA isolated from this screening, the miRNA mir-16, mir 15b, mir-106a, mir-17-5p were identified as being expressed in germinal cells, but presented also a ubiquitous expression.

Mir34C alone appears to present a restricted expression to germinal cells. This result was confirmed by both Northern Blot and RT-PCR analysis.

The sequence of the human pre-microRNA-34c and of the mature microRNA-34c are presented here as SEQ ID N°1 and SEQ ID N°2, respectively. Preferentially the homolog sequence has at least 90 % of homology with the SEQ ID N°2.

To induce the differentiation towards germinal cells of chicken and mammalian stem cells, or chicken and mammalian somatic cells, the use of a two step process allowing the successive expression of a Vasa gene and of a micro-RNA 34c, or a DNA sequence encoding for said microRNA, gives best results than the expression of a Vasa gene alone.

Any miRNA-34c from any species can be used in the purpose of the invention. The miRNA-34c may be from the same species than the host cells, or from a different species than the host cells.

A DNA sequence encoding for the microRNA-34c may be integrated into the same genetic construction comprising the Vasa gene, or may be part of another genetic construction. In particular said encoding sequence preferably have its own promoter and termination sequences.

In the case of both coding sequences are part of two different cassettes, both cassettes can be introduced concomitantly or separately into the host cells.

As previously specified, the exogenous genetic constructions according to the invention comprise an expression promoter that is functional in the host cells. This promoter can be selected among ubiquitous promoters and tissue-specific promoters.

Ubiquitous promoters, functional in any type of cells, are for example: promoters of the genes CMV, TK, SV40, beta-actine, PGK, E2Fa....

Tissue-specific promoters can be selected among the following:
- Promoters specifically functional in embryonic stem cells :
   o Oct4 (mammals), 1P06 (chicken), Nanog, Rexl, Utfl
   o Sox1, Sox2, Sox3, Nestin
   o Gcnf, Eomes
   o Ens1
- Promoters specifically functional in somatic cells.

Preferentially, the promoter is inducible and can be induced easily by the addition of an induction factor into the growth medium. These promoters are well known in the art and can be retrieved with their sequences in usual NCBI or EMBL gene databases, wich are incorporated herein by reference. For example an inducible promoter is the tetracycline- inducible promoter.

A specific goal of this invention is to obtain transgenic germinal cells that express at least one exogenous gene of interest.

Therefore, according to a preferred embodiment of the invention, the stem cells or somatic cells are transformed with at least one exogenous gene of interest.

In a first aspect of the invention, the Vasa gene and the gene(s) of interest are comprised in the same genetic construction.

In another aspect of the invention, the Vasa gene and the gene(s) of interest are part of two or more different genetic constructions. The cassettes can be introduced concomitantly or separately into the host cells.

The transformation of the cells may be realized according to any technique known by the man skilled in the art, keeping in mind to use the more appropriate technique according to the cellular type to be transformed.

According to a preferred aspect of the invention, transformation of the cells is performed before the step of culture allowing the differentiation into germinal cells to occur.

A gene of interest is any gene whose expression is desired by the man skilled in the art. Gene products expressed by the gene of interest can be translated or non-translated gene products such as proteins, RNA, siRNA, ribozymes. In the present goal of the invention, a preferred gene of interest is chosen among genes encoding for proteins and more particularly Reproductive Hormones, Growth Hormone, Antibodies or fragments thereof, Growth Factors, Membrane Receptors, or Virus Receptors.

Said gene of interest is expressed under the control of a promoter. Said promoter can be ubiquitous or tissue-specific, and optionally inducible.

In a preferred aspect of the invention, the promoter controlling the expression of the gene of interest is specifically functional in germinal cells. Germinal cells-specific promoters are for example:
- promoter of the Vasa gene
- promoter of genes Nanoz, Dazl, Pumilio, Staufen, Tudor, ...
- promoter of genes Stella, Fragilis...
- promoter of Blimp1 ; this promoter is also specifically functional in Lymphocytes B but at another stage of development.

The appropriate medium is determined in function of the cellular type of the transformed cells. The appropriate medium allowing differentiation towards germinal cells is characterized by a reduction in serum concentration and absence of growth factors, which in contrast are present in the proliferation medium.

In the present defined conditions, the highest percentage of germinal differentiation is obtained after 2 to 5 days of pacing the cells into the appropriate medium. Further addition of specific growth factors such as BNDF, BMPs could improve such kinetic and efficiency of germinal cell differentiation.

Preferentially, the appropriate medium of culture contains a differentiation induction factor. Examples of such differentiation induction factors are:
- A factor belonging to the BMP family, such as BMP4, BMP8, BMP7, BMP2, BMP15...
- a factor belonging to the TGFβ family, such as TGFβ, nodal, activine, GDF9, ...
- Ligands of the receptor c-Ret, BDNF, NT3, NT4 (neurotrophin-3, -4)...
Concentrations of these factors are easily determined by the man skilled in the art.

In an another aspect, the invention is related to isolated germinal cells obtainable, and to isolated germinal cells obtained by the method according to the invention.

Depending on the initial stem cells or somatic cells induced into differentiation, these germinal cells are from any species and in particular from chicken or mammal.

These cells can be characterized by the expression of specific markers and/or by their ability to colonize the gonads when injected into an embryo.

In a preferred aspect of the invention, germinal cells such as obtainable by the invention express a product encoded by at least one exogenous gene of interest. In particular said gene product is a protein of interest.

The present invention is also related to non-mammal embryonic stem cells transformed with an exogenous genetic construction comprising at least one Vasa gene.

In an another aspect, the invention is related to mammal embryonic or adult stem cells transformed with an exogenous genetic construction comprising at least one Vasa gene and one miRNA-34c. Preferentially the mammal cells are non-human, and more preferentially are murine cells. Preferentially the stem cells are from embryonic origin, and more preferentially are murine ES cells.

In an another aspect, the invention is related to somatic cells, i.e. non-stem, non-germinal cells, transformed with an exogenous genetic construction comprising at least one Vasa gene and one miRNA-34c.

Somatic cells can be cells from any species and in particular human cells, murine cells, chicken cells. An example of somatic immortalized human cells is Hela cells.

The present invention is also related to a method for obtaining a chimeric non-human animal, wherein:
- germinal cells obtained with the method of the invention, or
- germinal cells obtained after differentiation of transformed stem cells or somatic cells cells according to the invention,
are injected into an embryo.

A chimeric animal or chimera is an animal that has two or more different populations of genetically distinct cells; here the somatic cells originate from an embryo into which germinal cells, obtained *in vitro,* have been injected, these cells being genetically different than the cells of the receptor embryo. Preferentially the germinal cells injected into the embryo are from the same species than the receptor embryo.

Production of chimeric animals is a well-known technique that has been developed in the last twenty years.

Another object of the present invention is a chimeric non-human animal obtained by the method according to the invention.

In a particular aspect of the invention, the chimeric non human animal obtained by the method according to the invention expresses at least one gene product encoded by at least one exogenous gene of interest.

Another object of the invention are progenies of the chimeric animals according to the invention, wherein at least one cell of said animal comprises a vasa gene or a vasa gene and a miRNA-34c, and/or express a product encoded by at least one exogenous gene of interest.

### DRAWINGS

**Figure 1****:** Table of percentages of sequence homology between Vasa genes from different species.
**Figure 2****:** Expression profiles of GFP/Cvh negative, GFP/Cvh median and GFP/Cvh High cESC. Levels were assessed by Quantitative RT-PCR on the different subpopulations. Value 1 is given arbitrary to the level in the control GFP/Cvh negative cESC population. Each sample was run on duplicate. 5 independent experiments gave similar results.
**Figure 3****:** Differentiation kinetic of GFP/Cvh Negative and GFP/Cvh High CESC.
   (A) Schematic representation of the experiment.
   (B-C) Expression levels during the 5 days differentiation process. 0,5x10⁶ cells were plated in 10cm dish and cultivated in differentiation medium with a low serum concentration (5%) and without proliferation cytokines for 5 days. Expression levels of germ associated genes were assessed by Quantitative RT-PCR. Value 1 was given, for each gene, to the levels at T0. Each sample was run on duplicate. 2 independent experiments gave the same results.

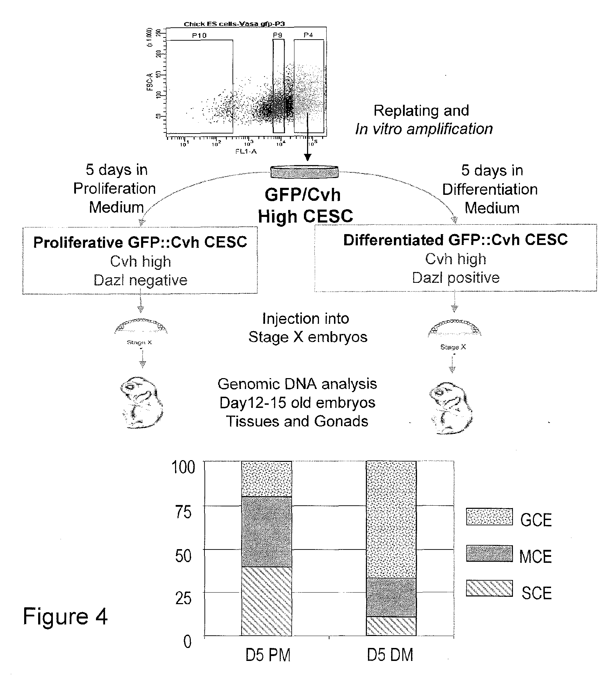

**Figure 4****:** Proliferative and differentiated GFP/Cvh cESC got a different colonization potential of host embryos.
   (A) Schematic representation of the experiment.
   (B) Distribution of the chimeric embryos obtained by injection of proliferative and differentiated GFP/Cvh CESC. Somatic chimeras (SCE) display a specific signal in Kidney and/or Heart. Mixed somatic and germ chimera (MCE) display GFP signals in Heart and/or Kidney and in Gonads. Lastly, Germline chimeras (GCE) display a specific GFP signal in the gonad exclusively. Quantative PCR on genomic DNA of each organ of each embryo was run in triplicate.

### EXAMPLES

### Example 1: Transformation of chicken embryonic stem cells with a genetic construction comprising a chicken Vasa gene and differentiation of cells towards germinal cells

Fertilised eggs from White Leghorn and Red-Naked neck hens were purchased from local breeder and incubated in a humidified incubator at 37°C.

cESC cells were maintained and transfected in proliferative medium (PM) as described in (Pain et al., 1996; Pain et al., 1999). Stable clones were obtained after daily addition of neomycin at 200µg/mL for 8 days.

### a) Exogenous expression of Cvh induced germcell associated gene expression and a loss of pluripotency associated gene expression

A fusion GFP::Cvh construct was introduced into the cESC. After transfection and drug selection, clones were obtained, pooled and cells FACS sorted (FACS Vantage TM SE Option Diva equipped with a Laser COHERENT ® EntrepriseTM IIC (488 nm and UV 351-364 nm)). Around 0.5% of cells were highly GFP positive.

No significant morphological differences can be detected between the two GFP::Cvh High and GFP::Cvh Negative cESC subpopulations.

The GFP::Cvh positive cells can be amplified *in vitro* under proliferative conditions and easily sorted again for further enrichment.

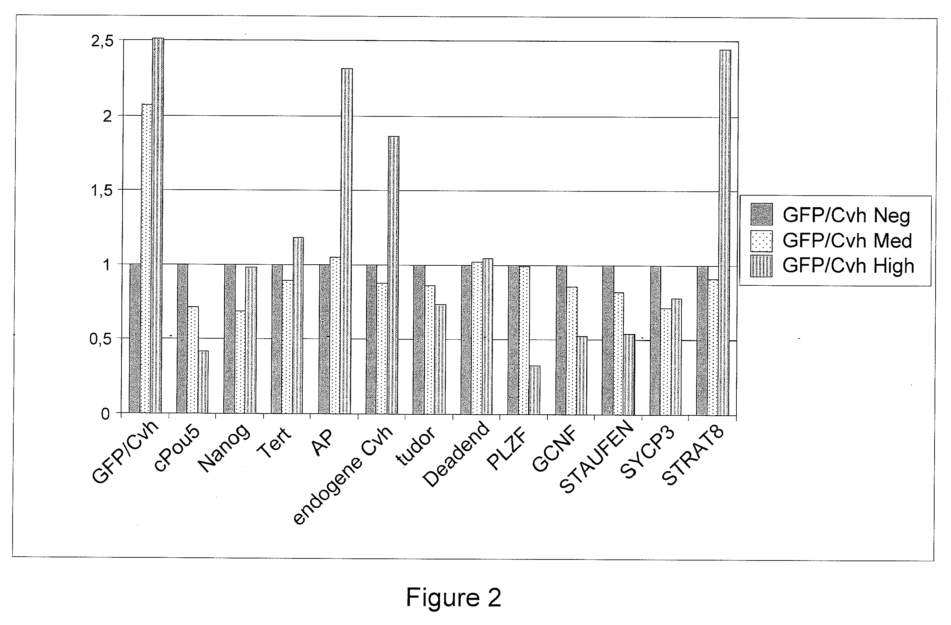

Figure 2 shows the gene expression level that was assessed by quantitative RT-PCR in the three cell sorted populations:
- GFP::Cvh High
- GFP::Cvh Median
- GFP::Cvh Negative cells.

It appears that the expression of cPouV (also called 1P06; the sequence is disclosed as SEQ ID N°1 in patent application PCT/EP2007/052572) is strongly downregulated in a dose dependent manner in cESC expressing Cvh up to a 60% decrease for the GFP::Cvh high cESC compared to the GFP::Cvh negative cESC.

This downregulation is concomitantly observed with an induction of some specific germ cell markers such AP, Strat8 and also the endogenous Cvh expression.

However, in these conditions, the expression of cDazl, a very specific marker of germ cells, remains undetectable in all GFP::Cvh sub-populations.

In conclusion, high exogenous expression of Cvh is supported by the cESC and leads to gene expression modifications, in particular a strong down regulation of pluripotent associated genes.

### b) Over-expressing Cvh cESC are able to differentiate into Dazl expressing germ cells

GFP::Cvh High and GFP::Cvh Negative cESC were plated in an appropriate Differentiation medium, without growth factors and cytokines, in the presence of a reduced serum concentration (5%).

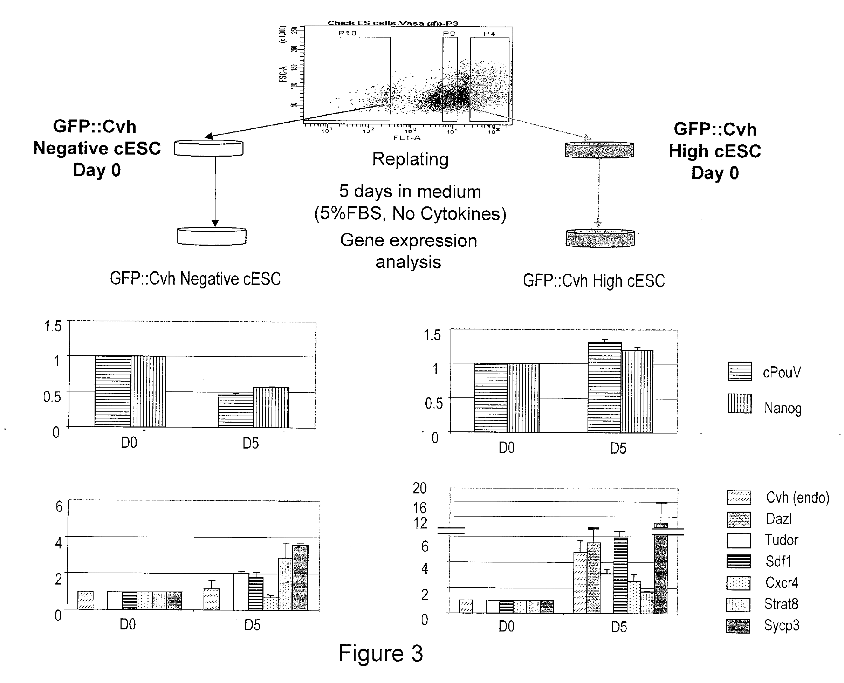

Under these conditions, the GFP::Cvh negative cells differentiate normally as observed by a decrease of the pluripotency associated gene expression such as cPouV, cNanog and cSox2. A moderate increase of expression of germ cell markers is observed, cDazl expression remains undetectable and endogenous level of Cvh constant. This is shown in figure 3.

When platted in the same conditions, the GFP::Cvh high cESC show various changes in their pattern of expression, as shown in figure 3 :
- pluripotency associated genes remain expressed
- germ cell specific markers are dramatically induced including cDazl and endogenous Cvh
- strong significant induction of expression premeiotic marker such as Strat8 and Scyp3 are induced
- Interestingly, expression of Sdfl, known to be involved in the chick PGC guidance, is also strongly induced.

Morphologically, the GFP::Cvh high cESC platted in the differentiation medium show a typical round morphology of PGCs.

### Example 2 : Characterization of germline cells obtained after differentiation of chicken ES cells

### a) Injection of cESC into stage X embryo

In order to evaluate the in vivo potential of the GFP::Cvh High cESC, these cells, maintained either in the proliferative medium (PM) or in the differentiation medium (DM) were injected into recipient stage X embryos according to previously described procedure (Pain et al., 1996).

Recipient embryos were irradiated at 6 Gy (Cobalt source). A small window was made on the lateral part of the egg and shell membrane was removed. 500 to 2000 cells in 1 to 3 µl were injected into the subgerminal cavity using a 20 µl borosilicate micropipette. The window was closed with two layers of shell membrane, reinforced with one piece of Visulin (Hartmann N° 685721/3).

Eggs were incubated for 12-15 days.

### b) GFP::Cvh high cESC are able to colonize the embryonic gonads with a high efficiency

Embryos were incubated for 13 to 15 days and the presence of GFP::Cvh positive cells analysed at both genomic and expression level in different embryonic removed somatic tissues such as heart and kidney and the gonads. The percentage of survival (around 8%) is identical between the embryos injected with mock medium or with these cells.

By genomic PCR analysis, the presence of GFP:Cvh signal was detected in 70% and 50% of the embryos respectively injected with the GFP:Cvh High cESC maintained in proliferation medium and differentiation medium conditions (Figure 4).

Non-injected control embryos do not provide any signal.

Three classes of chimera are distinguished :
- Somatic Chimeras embryos (SCE), when the signal is detected only in somatic tissues
- Mixed Chimeras embryos (MCE) when the signal is detected in both somatic tissues and gonads
- Germline chimeras embryos (GCE) when the signal is exclusively detected in the gonads of the injected embryos.

GFP::Cvh high cESC cells in DM condition exhibit a strong gonad colonization with the obtention of 86% MCE and 70% of GCE (Figure 4).

The same cells maintained in the proliferation medium do contribute only for 7% of GCE, 47% of MCE and 46% of SME.

This result clearly indicates the strong germinal tropism of the GFP::Cvh High cESC cultivated in appropriate conditions.

Beside this genomic analysis, in situ hybridization was performed on the one remaining gonad not used for genomic analysis and revealed the presence of GFP positive cells in the cortical zone corresponding to the localization of cDAzl positive germ cells.

In conclusion, these results demonstrate the ability of the Cvh modified cESC to be induced in germ cells able to colonize *in vivo* the gonad concomitantly with the lost of their somatic differentiation potential.

## Claims

1. Method for inducing *in vitro* the differentiation of stem cells or somatic cells towards germinal cells, comprising the following steps :
• cultivating said stem cells or somatic cells in a medium allowing their differentiation, and
• collecting the germinal cells from the culture medium,
wherein said stem cells or somatic cells are cells transformed with an exogenous genetic construction comprising at least a Vasa gene.

2. Method of claim 1 wherein the stem cells or somatic cells are chicken Embryonic Stem Cells (cESC).

3. Method of claim 2 wherein the stem cells or somatic cells are Chicken Embryonic Stem Cells that arose from the *in vitro* culture of pre-primitive streak (stage IX-XII) blastodermal cells.

4. Method of claim 1 wherein the stem cells or somatic cells are non-human mammalian embryonic stem cells.

5. Method of claim 1 wherein the stem cells or somatic cells are somatic cells.

6. Method according to claims 4 or 5 wherein the stem cells or somatic cells are transformed with a genetic construction comprising the microRNA-34c.

7. Method according to anyone of claims 1 to 6 wherein the stem cells or somatic cells are transformed with at least one exogenous gene of interest.

8. Method of claim 7 wherein said gene of interest is expressed under the control of a tissue-specific promoter functional in germinal cells.

9. Method according to anyone of claims 1 to 8 wherein the appropriate medium of culture contains a differentiation induction factor.

10. Method according to claim 9 wherein the induction factor is chosen among the group consisting of: a factor belonging to the BMP family, a factor belonging to the TGFβ family, ligands of the c-Ret receptor, BDNF, NT3 or NT4.

11. Germinal cells obtained by the method according to anyone of claims 1 to 10.

12. Germinal cells obtained by the method according to anyone of claims 7 or 8 wherein said cells express the product encoded by at least one exogenous gene of interest.

13. Non-mammal embryonic stem cells transformed with an exogenous genetic construction comprising at least one Vasa gene.

14. Non-human mammal embryonic or adult stem cells transformed with an exogenous genetic construction comprising at least one Vasa gene and at least one miRNA-34c.

15. Somatic cells transformed with an exogenous genetic construction comprising at least one Vasa gene and at least one miRNA-34c.

16. Method for obtaining a chimeric non-human animal wherein germinal cells according to claims 11 or 12, or obtained after differentiation of cells according to anyone of claims 13 to 15, are injected into an embryo.

17. Chimeric non-human animal obtained by the method according to claim 16.

18. Chimeric non human animal obtained by the method according to claim 16, wherein at least one cell of said chimeric animal expresses at least one product encoded by at least one exogenous gene of interest.

19. Progenies of the chimeric animals according to claims 17 or 18, wherein at least one cell of said animal comprises a vasa gene or a vasa gene and a miRNA-34c, and/or express a product encoded by at least one exogenous gene of interest.


SEQUENCE LISTING

<110>  INRA

<120>  METHOD FOR REPROGRAMING IN VITRO STEM CELLS AND SOMATIC CELLS
       INTO GERMINAL CELLS

<130>  67194 D25960

<160>  2

<170>  PatentIn version 3.3

<210>  1
<211>  77
<212>  RNA
<213>  Homo sapiens

<400>  1
agucuaguua cuaggcagug uaguuagcug auugcuaaua guaccaauca cuaaccacac      60

ggccagguaa aaagauu                                                     77


<210>  2
<211>  23
<212>  RNA
<213>  Homo sapiens

<400>  2
aggcagugua guuagcugau ugc                                              23